# EUROPÄISCHE PATENTSCHRIFT

(11) **EP 0 795 585 B1**
(45) Veröffentlichungstag und Bekanntmachung des Hinweises auf die Patenterteilung: **24.10.2001**
(21) Anmeldenummer: 97103860.9
(22) Anmeldetag: 07.03.1997
(51) Int. Cl.: C09B 61/00, A23L 1/275

(54) **Feinverteilte Carotinoid- und Retinoidsuspensionen und Verfahren zu ihrer Herstellung**
Finely divided suspensions of carotenoids or retinoids and process to manufacture them
Suspensions finement divisées de caroténoides ou rétinoides et leur procédé de fabrication

(30) Priorität: 11.03.1996 DE 19609538
(43) Veröffentlichungstag der Anmeldung: 17.09.1997
(73) Patentinhaber: BASF AKTIENGESELLSCHAFT, 67056 Ludwigshafen (DE)
(72) Erfinder: Lüddecke, Erik, Dr., 67112 Mutterstadt (DE); Schweikert, Loni, Dr., 67122 Altrip (DE)
(74) Vertreter: Isenbruck, Günter, Dr.

(56) Entgegenhaltungen:
- EP-A- 0 055 817
- EP-A- 0 065 193
- EP-A- 0 239 949
- EP-A- 0 479 066
- WO-A-91/16068
- FR-A- 2 281 961
- ULLMANN'S ENCYCLOPEDIA OF INDUSTRIAL CHEMISTRY 5TH ED. Bd. A9, 1987, WEINHEIM, Seite 318
- 'Alginate, Alginsäure' RÖMPP CHEMIE LEXIKON Bd. 9, 1989, STUTTGART, Seite 95, 2301
- BUNDESVERBAND DER PHARMAZEUTISCHEN INDUSTRIE (HRSG.): "Rote Liste 1993: Roaccutan Nr. 31 228", EDITIO CANTOR, AULENDORF, , Band , Nr. , Seiten -

## Beschreibung

Die Erfindung betrifft feinverteilte Carotinoid- und Retinoidsuspensionen, die beispielsweise zum Färben von Lebens- und Futtermitteln verwendbar sind, und ein Verfahren zu ihrer Herstellung.

Carotinoide sind in der Natur weit verbreitete Farbpigmente mit einem gelben bis roten Farbton. Sie verleihen vielen Nahrungsmitteln eine charakteristische Färbung. Wichtige Vertreter der Stoffklasse der Carotinoide sind β-Carotin, β-apo-8'-Carotinal, Cantaxanthin und Citranaxanthin. Diese Farbstoffe sind synthetisch herstellbar und können als Ersatz für künstliche Farbstoffe zur Färbung von Lebens- und Futtermitteln dienen. Sie finden zudem in der pharmazeutischen Industrie Einsatz, beispielsweise wegen ihrer pro-Vitamin-A-Aktivität.

Um als Farbstoffe in Lebens- und Futtermitteln, sowie in der pharmazeutischen Industrie einsetzbar zu sein, müssen die Carotinoide bzw. Retinoide in einer feinverteilten Form vorliegen, da sie in Wasser unlöslich sind und in Fetten und Ölen eine nur geringe Löslichkeit aufweisen. Zudem weisen die Carotinoide und Retinoide eine hohe Oxidationsempfindlichkeit auf.

Verschiedene Verfahren zur Herstellung von feinverteilten Carotinoiden und Retinoiden sind bekannt.

In US 5,091,188 und 5,091,187 sind Phospholipid-beschichtete Mikrokristalle beschrieben, die injizierbare Formulierungen von wasserunlöslichen pharmazeutischen Wirkstoffen darstellen. Es werden einer Reihe von wasserunlöslichen pharmazeutischen Wirkstoffen, wie Oxytetracyclin (OTC), Erythromycin, Albendazol, Nitroscanat oder Alfaxalon mit Hilfe von Phospholipiden, wie Lecithin in eine feinteilige Dispersion überführt. Unter den vorgeschlagenen Verfahren zur Herstellung der Dispersionen wird die Lösungsverdünnung beschrieben, bei der Lösungen von Lipid und wasserunlöslichem pharmazeutischem Wirkstoff in einem mit Wasser mischbaren organischen Lösungsmittel wie Ethanol hergestellt werden. Diese Lösungen werden in ein wäßriges Medium unter starkem Rühren gegeben, wobei sich die Mikrokristallite bilden. Eine Verwendung von Carotinoiden oder Retinoiden ist nicht erwähnt.

In der EP-0 065 193 ist ein Verfahren zur Herstellung von feinverteilten, pulverförmigen Carotinoid- bzw. Retinoidpräparaten beschrieben, wobei man das Carotinoid bzw. Retinoid in einem flüchtigen, mit Wasser mischbaren, organischen Lösungsmittel bei Temperaturen zwischen 50°C und 200°C, gegebenenfalls unter erhöhtem Druck, innerhalb einer Zeit von weniger als 10 Sek löst, aus der erhaltenen molekulardispersen Lösung sofort durch schnelles Mischen mit einer wäßrigen Lösung eines quellbaren Kolloids bei Temperaturen zwischen 0°C und 50°C das Carotinoid in kolloid-disperser Form ausfällt und die erhaltene Dispersion in an sich bekannter Weise von dem Lösungsmittel und dem Dispergiermedium befreit. Neben einem quellbaren Kolloid können ein Weichmacher und gegebenenfalls Stabilisatoren eingesetzt werden. Lecithin wird als verwendbarer Stabilisator aufgeführt. Es wird aufgeführt, daß der Feinheitsgrad durch die Wahl der der Carotinoidlösung zugesetzten Stabilisatoren gesteuert werden kann.

EP-A-0 239 949 betrifft ein Verfahren zur Herstellung von feinverteilten, pulverförmigen Carotinoidpräparaten. Das Verfahren entspricht dem in EP-A-0 065 193 beschriebenen Verfahren, lediglich das quellbare Kolloid wird durch Milch ersetzt. Die Milch soll dabei die Wirkung des quellbaren Kolloids ersetzen. Die Milch wird zur Bildung des mikrodispersen Wirkstoffmikronisatzs eingesetzt, wobei die Milch eine dem quellbaren Kolloid vergleichbare Wirkung zeigt, obwohl sie denaturieren und koagulieren kann. Zudem wird angegeben, daß Lecithin zusätzlich als Stabilisator gegen oxidativen Abbau eingesetzt werden kann.

Aus der EP-A-0 479 066 ist ein Verfahren zur kontinuierlichen Herstellung von β-Carotin-Solubilisaten durch Erhitzen von β-Carotin zusammen mit einem Emulgator bis zur Lösung, Abkühlung der homogenen Lösung auf unter 100°C durch Zugabe von Wasser und anschließende Einstellung der gewünschten Endkonzentration bekannt. Als verwendbare Emulgatoren werden ethoxilierte Triglyceride von Fettsäuren, ethoxilierte Sorbitanfettsäureester und ethoxilierte Monohydroxifettsäuren aufgeführt. Das Erhitzen des β-Carotins zusammen mit dem Emulgator erfolgt für einen Zeitraum von 17 bis 68 Sek. Die Solubilisate sind schutzkolloidfrei.

In der EP-B-0 055 817 ist ein Verfahren zur Herstellung stabiler injizierbarer β-Carotin-Solubilisate beschrieben. Dazu wird ein Emulgator auf eine Temperatur von 160 bis 180°C erhitzt und β-Carotin in die Schmelze eingetragen in einem Zeitraum von ca. 5 Minuten. Nach Lösen des β-Carotins wird die Lösung mit Wasser versetzt und auf 60 bis 80°C abgekühlt, wobei das Solubilisat entsteht. Als verwendbare Emulgatoren sind ethoxilierte Triglyceride von Fettsäuren, ethoxilierte Sorbitanfettsäureester und ethoxilierte Monohydroxifettsäuren aufgeführt. Die Solubilisate sind schutzkolloidfrei.

Eine Aufgabe der vorliegenden Erfindung ist die Bereitstellung von feinverteilten Carotinoid- und Retinoidsuspensionen, wobei auf ein Schutzkolloid verzichtet werden kann, sowie eines Verfahrens zu ihrer Herstellung.

Eine weitere Aufgabe der Erfindung ist die Bereitstellung eines Verfahrens zur Herstellung von feinverteilten Carotinoid- und Retinoidsuspensionen, wobei die Carotinoide oder Retinoide schonend behandelt werden.

Eine weitere Aufgabe der Erfindung ist die Bereitstellung von feinverteilten Carotinoid- und Retinoidsuspensionen, die physiologisch unbedenklich sind, sowie eines Verfahrens zu ihrer Herstellung.

Eine weitere Aufgabe der Erfindung ist die Bereitstellung von feinverteilten Carotinoid- und Retinoidsuspensionen und eines Verfahrens zu ihrer Herstellung, wobei der Farbton der erhaltenen Suspensionen variiert werden kann.

Eine weitere Aufgabe der Erfindung ist die Bereitstellung von feinverteilten Carotinoid- und Retinoidsuspensionen, die einen hohen Wirkstoffgehalt aufweisen und dünnflüssig sind, sowie eines Verfahrens zu ihrer Herstellung.

Die Aufgaben werden erfindungsgemäß gelöst durch

Ein Verfahren zur Herstellung von feinverteilten Carotinoid- oder Retinoidsuspensionen, bestehend aus Wasser, einem flüchtigen, mit Wasser mischbaren organischen Lösungsmittel, einem Carotinoid oder Retinoid, mindestens einem physiologisch verträglichen Emulgator, ausgewählt aus Lecithin, einem Fettsäuresalz, einem Mono-, Di- oder Triglyderid von C₁₂-C₁₈-Fettsäuren oder aliphatischen, gegebenenfalls acetylierten, Polycarbonsäuren, gegebenenfalls verestert mit Fruchtsäuren, eine Zuckerfettsäureester, oder einem Polyglycerinester von C₁₂-C₁₈-Fettsäuren, und gegebenenfalls einem Antioxidationsmittel, durch Lösen des Carotinoids oder Retinoids in dem flüchtigen, mit Wasser mischbaren organischen Lösungsmittel bei einer Temperatur von 50°C bis 250°C, gegebenenfalls unter erhöhtem Druck, innerhalb einer Zeit von weniger als 10 Sekungen, und sofort darauf folgendes Mischen der Lösung mit einem wässrigen Medium bei einer Temperatur von 0 bis 90°C, wobei das Mischen mit dem wässrigen Medium in Gegenwart des mindestens einen physiologisch verträglichen Emulgators erfolgt.

Die Aufgaben werden zudem gelöst durch eine Carotinoid- oder Retinoidsuspension mit einer Teilchengröße kleiner 1 µm in einem wasserhaltigen Medium, dadurch gekennzeichnet, daß die Suspension besteht aus Wasser, einem flüchtigen, mit Wasser mischbaren organischen Lösungsmittel, einem Carotinoid oder Retinoid, mindestens einem physiologisch verträglichen Emulgator, ausgewählt aus Lecithin, einem Festtsäuresalz, einem Mono-, Di- oder Triglycerid von C₁₂-C₁₈-Fettsäuren oder aliphatischen, gegebenenfalls acetylierten, Polycarbonsäuren, gegebenenfalls verestert mit Fruchtsäuren, einem Zuckerfettsäureester, oder einem Polyglycerinester von C₁₂-C₁₈-Fettsäuren, und gegebenenfalls einem Antioxidationsmittel.

Die Carotinoidsuspension oder das feinverteilte Carotinoidpulver können als Färbemittel für Lebens- und Futtermittel verwendet werden.

### CAROTINOIDE UND RETINOIDE

Das erfindungsgemäße Verfahren wird zur Herstellung von feinverteilten Carotinoid- und Retinoidsuspensionen verwendet.

Beispiele für erfindungsgemäß verwendbare Carotinoide sind die bekannten, zugänglichen, natürlichen oder synthetischen Vertreter dieser Verbindungsklasse, beispielsweise Carotin, Lycopin, Bixin, Zeaxanthin, Cryptoxanthin, Citranaxanthin, Lutein, Canthaxanthin, Astaxanthin, β-Apo-4'-Carotinal, β-Apo-8'-Carotinal, β-Apo-12'-Carotinal, ß-Apo-8'-Carotinsäure sowie Ester von hydroxi- oder carboxihaltigen Verbindungen dieser Gruppe, beispielsweise niedere Alkylester, vorzugsweise Methyl- und Ethylester. Diese Verbindungen sind beispielsweise als farbgebende Mittel gut verwendbar. Besonders bevorzugt sind die technisch erhältlichen Vertreter, wie β-Carotin, Canthaxanthin, β-Apo-8'-Carotinal und β-Apo-8'-Carotinsäureester.

Ebenfalls verwendbar sind Retinoide, beispielsweise All-Trans-Retinsäure, 13-cis-Retinsäure und die Ester und Amide dieser Säuren. Verwendbare Verbindungen dieser Art sind beschrieben von D.L. Newton, W.R. Henderson und M.B. Sporn in Cancer Research 40, 3413-3425.

### LÖSUNGSMITTEL

Im erfindungsgemäßen Verfahren wird ein flüchtiges mit Wasser mischbares organisches Lösungsmittel verwendet, in dem die eingesetzten Carotinoide oder Retinoide bei erhöhter Temperatur löslich sind. Jedes geeignete Lösungsmittel kann erfindungsgemäß verwendet werden, vorzugsweise werden wassermischbare, thermisch stabile, flüchtige, nur Kohlenstoff, Wasserstoff und Sauerstoff enthaltende Lösungsmittel verwendet Beispiele geeigneter Lösungsmittel sind Alkohole, Ether, Ester, Ketone und Acetale und deren Gemische. Vorzugsweise werden Ethanol, n-Propanol, Isopropanol, 1,2-Butandiol-1-methylether, 1,2-Propandiol-1-n-propylether oder Aceton oder ein Gemisch von zweien oder mehreren davon verwendet.

Vorzugsweise werden solche Lösungsmittel verwendet, die zu mindestens 10 Volumen-% wassermischbar sind, einen Siedepunkt von unterhalb 200°C aufweisen und gegebenenfalls weniger als 10 Kohlenstoffatome in ihrer Struktur aufweisen.

Das Carotinoid oder Retinoid wird in dem beim Lösen verwendeten flüchtigen, mit Wasser mischbaren organischen Verdünnungs- oder Lösungsmittel eingesetzt.

### EMULGATOREN

Zur Herstellung der erfindungsgemäßen feinverteilten Carotinoid- und Retinoidsuspensionen werden erfindungsgemäß physiologisch verträgliche Emulgatoren eingesetzt

Der Ausdruck "physiologisch verträglich" bedeutet hierbei, daß die Emulgatoren bei der Verabreichung in üblichen Mengen an Menschen oder Tiere physiologisch unbedenklich sind und nicht zu einer Schädigung des Organismus führen. Das gilt insbesondere bei oraler oder intramuskulärer Verabreichung.

Nachstehend sind die erfindungsgemäß verwendbaren Emulgatoren aufgeführt.

Gemäß einer bevorzugten Ausführungsform der Erfindung kann zur Herstellung der erfindungsgemäßen feinverteilten Carotinoid- und Retinoidsuspensionen Lecithin als Emulgator eingesetzt werden. Lecithine sind auch unter dem Namen Phosphatidylcholine bekannt und gehören zur Gruppe der Glycero-Phospholipide, die sich aus Fettsäuren, Glycerin, Phosphorsäure und Cholin durch Veresterung bilden.

Erfindungsgemäß verwendbar sind alle geeigneten Phosphatidylcholine, insbesondere die in der Natur vorkommenden Phosphatidylcholine, die Derivate der 1,2-Diacyl-sn-glycerin-3-phosphorsäuren sind. - Verwendbar sind Phosphatidylcholine mit gleichen oder unterschiedlichen Fettsäureresten sowie Gemische davon.

Eine Lecithinfraktion aus Sojabohnen enthält beispielsweise Fettsäurereste von Palmitinsäure, Stearinsäure, Palmitoleinsäure, Oleinsäure, Linolsäure und Linolensäure.

Einsetzbar sind Phosphatidylcholine mit sowohl ungesättigten als auch gesättigten Fettsäureresten.

Gemäß einer besonders bevorzugten Ausführungsform der Erfindung werden zur Herstellung der erfindungsgemäßen feinverteilten Carotinoid- und Retinoidsuspensionen partiell hydrolisierte Lecithine verwendet, insbesondere solche, die einen Gehalt an Lysophospholipiden von 10 - 15 Gew.-% aufweisen. Ein Beispiel für ein solches Lecithin bzw. Lecithingemisch ist Emulfluid® E von der Lucas Meyer GmbH.

Gemäß einer Ausführungsform der Erfindung kann zur Herstellung der erfindungsgemäßen feinverteilten Carotinoid- und Retinoidsuspensionen ein Mono-, Di- oder Triglycerid einer aliphatischen Di- oder Polycarbonsäure als Emulgator eingesetzt werden. Die Di- oder Polycarbonsäure kann Hydroxylgruppen aufweisen, die ggf. durch Acetylreste substituiert sein können. Beispiele verwendbarer Säuren sind Citronensäure oder Weinsäure. Beispiele für verwendbare Säureglyceride sind Citronensäureester eines Mono- oder Diglycerids (beispielsweise Acidan N12® von Grinstedt), und Diacetylweinsäureester von Monoglyceriden (Diacetyltartraric esters of monoglycerides, DATEM, beispielsweise Panodan TR® von Grinstedt).

Gemäß einer Ausführungsform der Erfindung kann zur Herstellung der erfindungsgemäßen feinverteilten Carotinoid- und Retinoidsuspensionen ein Zuckerfettsäureester als Emulgator eingesetzt werden. Dabei können physiologisch verträgliche Fettsäuren verwendet werden, wie Laurinsäure, Palmitinsäure, Stearinsäure, oder einfach oder mehrfach ungesättigte Fettsäuren, wie Linolsäure oder Linolensäure. Der Zuckerrest kann ein beliebiger geeigneter Zuckerrest sein, vorzugsweise ein Ascorbylrest. Ein Beispiel eines verwendbaren Zuckerfettsäureesters ist Ascorbylpalmitat.

Weiterhin verwendbar sind Salze von physiologisch verträglichen Fettsäuren, wie sie vorstehend beschrieben sind, sowie Mono- und Diglyceride dieser Fettsäuren. Die Mono- und Diglyceride dieser Fettsäuren können ggf. mit Fruchtsäuren verestert sein.

Gemäß einer Ausführungsform der Erfindung können weiterhin Polyglycerinester dieser Fettsäuren verwendet werden.

Vorzugsweise wird Lecithin als Emulgator zur Herstellung der erfindungsgemäßen feinverteilten Carotinoid- und Retinoidsuspensionen eingesetzt.

Die erfindungsgemäß verwendbaren Emulgatoren, insbesondere Lecithin, sind physiologisch unbedenklich und können somit in pharmazeutischen Zusammensetzungen verwendet werden.

Darüber hinaus wurde erfindungsgemäß gefunden, daß die erfindungsgemäß verwendbaren Emulgatoren, insbesondere das gemäß einer Ausführungsform verwendete Lecithin, eine sehr rasche emulgierende Wirkung aufweisen, was ihre Verwendung in einem Hochgeschwindigkeitsverfahren zur Herstellung von feinverteilten Carotinoid- und Retinoidsuspensionen ermöglicht.

Erfindungsgemäß wurde gefunden, daß bei Verwendung der erfindungsgemäßen Emulgatoren, insbesondere von Lecithin bei der Herstellung von feinverteilten Carotinoid- und Retinoidsuspensionen auf die Verwendung eines Schutzkolloids verzichtet werden kann. Der Verzicht auf ein Schutzkolloid führt zu flüssigen Carotinoid- und Retinoidsuspensionen mit einer geringeren Viskosität und einem höheren Wirkstoffgehalt. Zudem wird, insbesondere für pharmazeutische Anwendungen, die Anzahl der Bestandteile der Suspensionen vermindert, so daß weniger störende Verbindungen vorliegen, die einen Einfluß auf die Wirksamkeit der Carotinoide und/oder Retinoide haben können.

Der erfindungsgemäß verwendete Emulgator, insbesondere das Lecithin, kann im erfindungsgemäßen Verfahren im wäßrigen Medium und/oder im organischen Lösungsmittel vorliegen. So kann der erfindungsgemäß verwendete Emulgator, insbesondere das Lecithin, entweder in der vor dem Lösen hergestellten Suspension des Carotinoids im mit Wasser mischbaren organischen Lösungsmittel enthalten sein oder wahlweise im mit Wasser mischbaren organischen Lösungsmittel, das bei hoher Temperatur vorliegt. Vorzugsweise wird der erfindungsgemäß verwendete Emulgator, insbesondere das Lecithin, sowohl in die organische Carotinoid- und Retinoidsuspension gegeben, als auch in das wäßrige Medium.

Das Mengenverhältnis von erfindungsgemäß verwendetem Emulgator, insbesondere von Lecithin, zu Carotinoid oder Retinoid kann beliebig gewählt werden, solange feinverteilte Carotinoid- und Retinoidsuspensionen erhalten werden, die stabil sind.

Vorzugsweise beträgt das Verhältnis von erfindungsgemäß verwendetem Emulgator, insbesondere von Lecithin, zu Carotinoid oder Retinoid in der Suspension 0,1 bis 5 (Verhältnis der Gewichtsteile). Besonders bevorzugt beträgt das Verhältnis 0,5 bis 2, insbesondere 0,7 bis 1.

Durch eine Veränderung des Mengenverhältnisses von erfindungsgemäß verwendetem Emulgator, insbesondere von Lecithin, zu Carotinoid oder Retinoid kann weiterhin der Farbton der erhaltenen Carotinoid- und Retinoidsuspensionen verändert werden. Bei hohen Anteilen an erfindungsgemäß verwendetem Emulgator, insbesondere von Lecithin, beispielsweise bei einem Gewichtsverhältnis von 2:1 von erfindungsgemäß verwendetem Emulgator, insbesondere von Lecithin, zu Carotin, erhält man eher gelbstichige Produkte, wobei im Falle von Carotin und Lecithin als Emulgator dieses im Lecithin zumindest teilweise gelöst vorliegt. Bei niedrigeren Anteilen von erfindungsgemäß verwendetem Emulgator, insbesondere von Lecithin, zu Carotinoid, wie 0,75:1, bleibt beispielsweise β-Carotin partikulär. Der Farbton der Suspension ist in diesem Fall rot-bräunlich.

Diese Variierbarkeit des Farbtons ist für den Einsatz der Carotinoid- und Retinoidsuspensionen der vorliegenden Erfindung als Lebensmittelfarbstoff von Vorteil, da je nach spezifischem Einsatzgebiet der Farbton einfach angepaßt werden kann.

Weiterhin ermöglicht der Verzicht auf Schutzkolloide in den Carotinoid- und Retinoidsuspensionen die Herstellung von dünnflüssigen Suspensionen mit hohen Wirkstoffgehalten, beispielsweise von bis zu 10 Gew.-%, bezogen auf die fertige Suspension. Dies vereinfacht die Verwendung und den Transport der Suspensionen, da sie selbst bei hohen Wirkstoffgehalten noch gut fließfähig und dosierbar sind.

Beispielsweise wird hierdurch der Einsatz von β-Carotin in der Getränkefärbung vereinfacht, da mit Flüssigdosiergeräten gearbeitet werden kann, die in Produktionsanlagen im allgemeinen präziser arbeiten als Feststoffdosiergeräte.

Ein weiterer Vorteil des Verzichts auf Schutzkolloide ist die bessere Verwendbarkeit der Suspensionen in pharmazeutischen Anwendungen. Beispielsweise können die erfindungsgemäß hergestellten Suspensionen als Injektionslösungen verwendet werden, beispielsweise in der Veterinärmedizin.

### ANTIOXIDATIONSMITEL

Im erfindungsgemäßen Verfahren kann dem organischen, mit Wasser mischbaren Lösungsmittel oder dem wäßrigen Medium, der fertigen Suspension des Carotinoids oder Retinoids wie auch den Carotinoiden und Retinoiden ein Antioxidationsmittel beigefügt werden. Das Antioxidationsmittel dient zur Erhöhung der Stabilität des Wirkstoffs gegen oxidativen Abbau. Vorzugsweise wird das Antioxidationsmittel, sofern es verwendet wird, gemeinsam mit den Carotinoiden oder Retinoiden in dem mit Wasser mischbaren organischen Lösungsmittel gelöst. Beispiele für verwendbare Antioxidationsmittel sind α-Tocopherol, t-Butylhydroxytoluol, t-Butylhydroxyanisol oder Ethoxyquine. Auch andere geeignete Antioxidationsmittel sind verwendbar.

### HERSTELLUNGSVERFAHREN

Die Carotinoid- und Retinoidsuspensionen werden erfindungsgemäß hergestellt durch Lösen des Carotinoids oder Retinoids in einem flüchtigen, mit Wasser mischbaren organischen Lösungsmittel bei einer Temperatur von 50°C bis 250°C, vorzugsweise 150 bis 200°C, gegebenenfalls unter erhöhtem Druck, innerhalb einer Zeit von weniger als 10 Sek, und sofort darauf folgendes Mischen der Lösung mit einem wäßrigen Medium bei einer Temperatur von 0 bis 90°C, vorzugsweise 2 - 50°C. Eine solche Vorgehensweise ist beispielsweise in der EP-B1-0 065 193 beschrieben. Das in dieser Patentschrift beschriebene Verfahren kann gemäß der vorliegenden Erfindung angewendet werden.

Der erfindungsgemäß verwendete Emulgator kann im wäßrigen Medium und/oder im organischen Verdünnungs- oder Lösungsmittel vorliegen. Vorzugsweise liegt der Emulgator, bevorzugt Lecithin, im wäßrigen Medium und im organischen Verdünnungs- oder Lösungsmittel vor.

Gemäß einer Ausführungsform der Erfindung wird das zur Herstellung der anfänglichen Suspension des Carotinoids oder Retinoids verwendete mit Wasser mischbare organische Lösungsmittel wie auch das wäßrige Medium, vorzugsweise Wasser, mit dem erfindungsgemäß verwendeten Emulgator, vorzugsweise Lecithin, versetzt.

Falls gewünscht, kann der erfindungsgemäß verwendete Emulgator auch im erhitzten organischen Lösungsmittel vorliegen.

Sodann wird gemäß einer Ausführungsform der Erfindung eine Suspension des Carotinoids oder Retinoids im mit Wasser mischbaren organischen Lösungsmittel hergestellt Vorzugsweise wird das gleiche mit Wasser mischbare organische Lösungsmittel verwendet, das im darauffolgenden Schritt als erhitztes organisches Lösungsmittel verwendet wird. Gemäß einer Ausführungsform der Erfindung beträgt die Konzentration des Carotinoids oder Retinoids in dieser Suspension 2 bis 40 Gew.-%, bezogen auf die Mischung. Diese Suspension oder das Carotinoid oder Retinoid wird sodann in erhitztem mit Wasser mischbarem organischem Lösungsmittel innerhalb eines Zeitraums von weniger als 10 Sek, vorzugsweise weniger als 5 Sek, besonders bevorzugt weniger als 2 Sek, insbesondere in Sekundenbruchteilen gelöst Dabei weist das erwärmte Lösungsmittel eine Temperatur von 50 bis 200°C, vorzugsweise 100 bis 180°C, besonders bevorzugt 140 bis 180°C auf. Nach der Zeit zum Lösen des Carotinoids oder Retinoids im erwärmten mit Wasser mischbaren organischen Lösungsmittel wird die Lösung sofort darauffolgend mit einem wäßrigen Medium bei einer Temperatur von 0 bis 50°C gemischt Vorzugsweise wird als wäßriges Medium Wasser verwendet. Beim Mischen der Lösung mit dem wäßrigen Medium entsteht eine feinteilige Dispersion des Carotinoids oder Retinoids.

Durch die sehr kurze Zeit, die zum Lösen des Carotinoids oder Retinoids erforderlich ist, wird das Carotinoid oder Retinoid nur für einen sehr kurzen Zeitraum einer erhöhten Temperatur ausgesetzt und sodann sofort wieder abgekühlt. Dieses ermöglicht eine sehr schonende Behandlung der Carotinoide und/oder Retinoide und vermindert die Gefahr einer Oxidation oder Zersetzung der Wirkstoffe. Im Vergleich zum erfindungsgemäßen Verfahren wird beispielsweise beim gemeinsamen Erhitzen von Wirkstoff und Lösungsmittel bis zur Lösung das Carotinoid oder Retinoid für einen wesentlich längeren Zeitraum einer erhöhten Temperatur ausgesetzt, verbunden mit der Gefahr der Oxidation und/oder thermischen Zersetzung des Wirkstoffs.

Gemäß einer bevorzugten Ausführungsform der Erfindung wird das Verfahren kontinuierlich in zwei Mischkammern durchgeführt. Dabei wird wahlweise zunächst eine Suspension des Wirkstoffs im organischen Lösungsmittel hergestellt und, beispielsweise mittels Pumpen, einer ersten Mischkammer zugeführt, der gleichzeitig das erwärmte organische Lösungsmittel zugeführt wird, so daß in der ersten Mischkammer das Lösen des Wirkstoffs im mit Wasser mischbaren organischen Lösungsmittel bei einer Temperatur von 50 bis 200°C erfolgt. Vorzugsweise beträgt die Wirkstoffkonzentration in dieser ersten Mischkammer 0,5 bis 10 Gew.-% bezogen auf die Lösung. Das Mischkammervolumen ist dabei vorzugsweise so bemessen, daß bei der gewählten Förderleistung der Pumpen für die Wirkstoffsuspension und das Lösungsmittel die Verweilzeit in der Kammer vorzugsweise weniger als 1 Sek beträgt.

Das Lösungsmittel wird vorzugsweise vor Eintritt in die Mischkammer über einen Wärmetauscher auf die gewünschte Temperatur gebracht, während die Wirkstoffsuspension durch Zuführung über eine thermisch isolierte Zuleitung bei einer Temperatur unterhalb 50°C gehalten wird. Vorzugsweise wird die Mischung in der ersten Mischkammer turbulent ausgeführt. Nach kurzer Verweilzeit, vorzugsweise von weniger als 1 Sek, tritt die Lösung in eine zweite Mischkammer ein, in der, beispielsweise über eine Pumpe, Wasser oder ein wäßriges Medium zugemischt wird und das Ausfällen der feinverteilten Carotinoid- und Retinoidsuspension bewirkt wird. Die feinteilige Wirkstoffsuspension kann sodann über eine weitere Leitung aus der zweiten Mischkammer ausgetragen werden und beispielsweise einem Vorratsgefäß zugeführt werden. Zur Erzielung einer möglichst hohen Wirkstoffkonzentration kann die Suspension im Kreis zur zweiten Mischkammer zurückgeführt werden.

Die Konzentration des Carotinoids oder Retinoids in der Suspension beträgt dabei vorzugsweise 0,1 bis 100 g/l.

Wird bei Drücken oberhalb 1 bar gearbeitet, so können Lösungsmittel bei Temperaturen oberhalb ihres Siedepunktes (bei Normaldruck) verwendet werden.

Aus der erhaltenen Suspension kann gemäß einer Ausführungsform der Erfindung ein pulverförmiges Präparat erhalten werden, beispielsweise gemäß dem in der DE-OS 2 534 091 beschriebenen Verfahren durch Sprühtrocknen oder durch Sprühkühlen oder durch Einhüllen der Teilchen, Abtrennen und Trocknen im Wirbelbett.

Das Verfahren zum Sprühtrocknen ist beispielsweise auch in der EP-B1-0 065 193 beschrieben.

Gemäß einer Ausführungsform der Erfindung kann aus der hergestellten Carotinoid- oder Retinoidsuspension das mit Wasser mischbare organische Lösungsmittel und/oder das wäßrige Medium zumindest teilweise entfernt werden, um eine konzentrierte Carotinoid- oder Retinoidsuspension herzustellen. Dabei kann die Konzentration des Carotinoids oder Retinoids in der Suspension 0,1 bis 100 g/l betragen.

Durch Einstellung geeigneter Mengenströme kann eine Carotinoid- oder Retinoidsuspension mit einer sehr geringen Wirkstoffpartikelgröße erhalten werden. Die Teilchengröße in der Carotinoid- oder Retinoidsuspension ist im wesentlichen < 1 *µ*m, vorzugsweise im Bereich von 0,01 bis 0,4 *µ*m, besonders bevorzugt im Bereich von 0,03 bis 0,2 *µ*m.

Beispielsweise kann bei einer Carotinkonzentration von 0,1 Gew.-%, bezogen auf die fertige Suspension, eine Suspension mit einer mittleren Wirkstoffpartikelgröße von 0,03 *µ*m erhalten werden. Dabei erscheint die Suspension als transparente Carotin-"Lösung". Wird der Wirkstoffgehalt höher eingestellt, so kann gemäß einer Ausführungsform der Erfindung die Partikelgröße zunehmen. Beispielsweise kann sie bei einer 0,4 Gew.-%igen Lösung im Mittel 0,06 *µ*m betragen. Durch Aufkonzentration der erhaltenen Suspension kann ein Wirkstoffgehalt von 1 bis 10 Gew.-%, bezogen auf die fertige Suspension, erreicht werden. Dieses kann beispielsweise durch schonendes Eindampfen bzw. durch Membranfiltration erfolgen. Die erhaltenen Suspensionen sind lagerstabil und haben eine nahezu unverändert hohe spezifische Farbstärke. Das eingesetzte organische Lösungsmittel kann gegebenenfalls aus dem Produkt entfernt werden, abhängig vom verwendeten Aufkonzentrationsschritt. Gemäß einer bevorzugten Ausführungsform wird als Lösungsmittel Isopropanol oder Ethanol verwendet und die Lösung des Carotinoids oder Retinoids erfolgt bei etwa 180°C in einem Überschuß an vorgeheiztem Alkohol, so daß eine homogene Lösung entsteht.

Beim Mischen mit dem als wäßriges Medium vorzugsweise verwendeten Wasser löst sich der Alkohol schlagartig im Wasser, wobei eine äußerst feinteilige Suspension des Carotinoids oder Retinoids entsteht.

Nachstehend wird die Erfindung anhand eines Ausführungsbeispiels näher erläutert.

### Beispiel 1

12,5 g β-Carotin werden in 490 g einer Lösung von 9 g Lecithin (Emulfluid® E, Lucas Meyer GmbH, hergestellt durch gezielte partielle Hydrolyse von nativem Lecithin, mit einem Gehalt an Lyso-Phospholipiden von 10-15 Gew.-%, einem HLB-Wert von 8 - 9) und 1,8 g d,1-α-Tocopherol in Isopropanol (Azeotrop) gelöst und in einer ersten Mischkammer mit 775 g Isopropanol (Azeotrop) gemischt, das im Wärmetauscher auf 220°C erhitzt wurde. Bei einer Dosierleistung von etwa 2 l/Std für die Suspension und 3 l/Std für das erhitzte Lösungsmittel beträgt die Verweilzeit in der Mischkammer 0,35 Sek. Dabei entsteht bei einer Tempeartur von 190°C eine molekulardisperse Lösung, die sodann einer zweiten Mischkammer zugeführt wird, in der sie unter turbulentem Vermischen mit 7800 g Wasser (Dosierleistung etwa 30 l/Std) versetzt wird. Dabei erfolgt die Bildung der feinteiligen Carotinsuspension, die in ein Auffanggefäß überführt wird. Im Auffanggefäß wird eine klare, orangefarbene Suspension des Carotins erhalten. Die Wirkstoffkonzentration betrug dabei 0,1 Gew.-% bezogen auf die fertige Suspension, die spezifische Farbstärke (Extinktion bei 1 cm Schichtdicke im Maximum der Absorptionsbande einer mit Wasser auf einen Wirkstoffgehalt von 5 ppm verdünnten Präparation) beträgt 0,72. Die Teilchengrößenanalyse mittels Photonen-Korrelations-Spektroskopie ergibt eine mittlere Teilchengröße von 70 nm.

### Beispiel 2

120 g β-Carotin werden in 540 g einer Lösung von 43 g Lecithin (Emulfluid® E, Lucas Meyer GmbH, siehe Beispiel 1) und 17 g d,1-α-Tocopherol in Isopropanol (Azeotrop) gelöst und in einer ersten Mischkammer mit 825 g Isopropanol (Azeotrop) gemischt, das im Wärmetauscher auf 220°C erhitzt wurde. Bei einer Dosierleistung von etwa 2 l/Std für die Suspension und 3 l/Std für das erhitzte Lösungsmittel beträgt die Verweilzeit in der Mischkammer 0,35 Sek. Dabei entsteht bei einer Tempeartur von 190°C eine molekulardisperse Lösung, die sodann einer zweiten Mischkammer zugeführt wird, in der sie unter turbulentem Vermischen mit 8800 g einer Lösung von 43 g Lecithin mit 10.400 g Wasser (Dosierleistung etwa 30 l/Std) versetzt wird. Dabei erfolgt die Bildung der feinteiligen Carotinsuspension, die in ein Auffanggefäß überführt wird. Im Auffanggefäß wird eine orangefarbene Suspension des Carotins erhalten. Die Wirkstoffkonzentration betrug dabei 1 Gew.-% bezogen auf die fertige Suspension, die spezifische Farbstärke (Extinktion bei 1 cm Schichtdicke im Maximum der Absorptionsbande einer mit Wasser auf einen Wirkstoffgehalt von 5 ppm verdünnten Präparation) beträgt 0,67. Die Teilchengrößenanalyse mittels Photonen-Korrelations-Spektroskopie ergibt eine mittlere Teilchengröße von 160 nm.

### Beispiel 3

12,5 g β-Carotin werden in 490 g einer Lösung von 1,8 g Zitronensäureester eines Mono/Diglycerids (Acidan N12® von Grinstedt) und 1,8 g d,1-α-Tocopherol in Isopropanol (Azeotrop) gelöst und in einer ersten Mischkammer mit 775 g Isopropanol (Azeotrop) gemischt, das im Wärmetauscher auf 220°C erhitzt wurde. Bei einer Dosierleistung von etwa 2 l/Std für die Suspension und 3 l/Std für das erhitzte Lösungsmittel beträgt die Verweilzeit in der Mischkammer 0,35 Sek. Dabei entsteht bei einer Tempeartur von 190°C eine molekulardisperse Lösung, die sodann einer zweiten Mischkammer zugeführt wird, in der sie unter turbulentem Vermischen mit 7800 g Wasser (Dosierleistung etwa 30 l/Std) versetzt wird. Dabei erfolgt die Bildung der feinteiligen Carotinsuspension, die in ein Auffanggefäß überführt wird. Im Auffanggefäß wird eine klare, orangefarbene Suspension des Carotins erhalten. Die Wirkstoffkonzentration betrug dabei 0,1 Gew.-% bezogen auf die fertige Suspension, die spezifische Farbstärke (Extinktion bei 1 cm Schichtdicke im Maximum der Absorptionsbande einer mit Wasser auf einen Wirkstoffgehalt von 5 ppm verdünnten Präparation) beträgt 0,66. Die Teilchengrößenanalyse mittels Photonen-Korrelations-Spektroskopie ergibt eine mittlere Teilchengröße von 80 nm.

### Beispiel 4

25 g β-Carotin werden in 950 g einer Lösung von 3,6 g Diacetylweinsäureester von Monoglyceriden (Panodan TR® von Grinstedt) und 3,6 g d,1-α-Tocopherol in Isopropanol (Azeotrop) gelöst und in einer ersten Mischkammer mit 1300 g Isopropanol (Azeotrop) gemischt, das im Wärmetauscher auf 220°C erhitzt wurde. Bei einer Dosierleistung von etwa 2 l/Std für die Suspension und 3 l/Std für das erhitzte Lösungsmittel beträgt die Verweilzeit in der Mischkammer 0,35 Sek. Dabei entsteht bei einer Temperatur von 190°C eine molekulardisperse Lösung, die sodann einer zweiten Mischkammer zugeführt wird, in der sie unter turbulentem Vermischen mit 15.400 g Wasser (Dosierleistung etwa 30 l/Std) versetzt wird. Dabei erfolgt die Bildung der feinteiligen Carotinsuspension, die in ein Auffanggefäß überführt wird. Im Auffanggefäß wird eine klare, orangefarbene Suspension des Carotins erhalten. Die Wirkstoffkonzentration betrug dabei 0,14 Gew.-% bezogen auf die fertige Suspension, die spezifische Farbstärke (Extinktion im Maximum der Absorptionsbande einer mit Wasser auf einen Wirkstoffgehalt von 5 ppm verdünnten Präparation) beträgt 0,72. Die Teilchengrößenanalyse mittels Photonen-Korrelations-Spektroskopie ergibt eine mittlere Teilchengröße von 220 nm.

### Beispiel 5

25 g β-Carotin werden in 290 g einer Lösung von 3,6 g Ascorbylpalmitat und 3,6 g d,1-α-Tocopherol in Isopropanol (Azeotrop) gelöst und in einer ersten Mischkammer mit 350 g Isopropanol (Azeotrop) gemischt, das im Wärmetauscher auf 220°C erhitzt wurde. Bei einer Dosierleistung von etwa 2 l/Std für die Suspension und 3 l/Std für das erhitzte Lösungsmittel beträgt die Verweilzeit in der Mischkammer 0,35 Sek. Dabei entsteht bei einer Tempeartur von 190°C eine molekulardisperse Lösung, die sodann einer zweiten Mischkammer zugeführt wird, in der sie unter turbulentem Vermischen mit 4150 g Wasser (Dosierleistung etwa 30 l/Std) versetzt wird. Dabei erfolgt die Bildung der feinteiligen Carotinsuspension, die in ein Auffanggefäß überführt wird. Im Auffanggefäß wird eine klare, orangefarbene Suspension des Carotins erhalten. Die Wirkstoffkonzentration betrug dabei 0,5 Gew.-% bezogen auf die fertige Suspension, die spezifische Farbstärke (Extinktion im Maximum der Absorptionsbande einer mit Wasser auf einen Wirkstoffgehalt von 5 ppm verdünnten Präparation) beträgt 0,69. Die Teilchengrößenanalyse mittels Photonen-Korrelations-Spektroskopie ergibt eine mittlere Teilchengröße von 120 nm.

Die hergestellten Suspensionen von β-Carotin sind physiologisch unbedenklich und über lange Zeit lagerstabil. Beim Herstellungsverfahren wird das verwendete β-Carotin sehr schonend behandelt, da es nur für eine sehr kurze Zeit (0,35 Sekunden) im erhitzten Lösungsmittel vorliegt. Der Farbton der feinverteilten Carotinoidsuspension variiert aufgrund der unterschiedlichen Teilchengröße, so daß der Farbton je nach Verfahrensführung eingestellt und variiert werden kann. Die Carotinoidsuspensionen weisen einen hohen Wirkstoffgehalt auf und sind dünnflüssig, so daß eine einfache Portionierung möglich ist, beispielsweise bei der Getränkeherstellung.

## Patentansprüche

1. Verfahren zur Herstellung von feinverteilten Carotinoid- oder Retinoidsuspensionen, bestehend aus Wasser, einem flüchtigen, mit Wasser mischbaren organischen Lösungsmittel, einem Carotinoid oder Retinoid, mindestens einem physiologisch verträglichen Emulgator, ausgewählt aus Lecithin, einem Fettsäuresalz, einem Mono-, Di- oder Triglycerid von C₁₂-C₁₈-Fettsäuren oder aliphatischen, gegebenenfalls acetylierten, Polycarbonsäuren, gegebenenfalls verestert mit Fruchtsäuren, eine Zuckerfettsäureester, oder einem Polyglycerinester von C₁₂-C₁₈-Fettsäuren, und gegebenenfalls einem Antioxidationsmittel, durch Lösen des Carotinoids oder Retinoids in dem flüchtigen, mit Wasser mischbaren organischen Lösungsmittel bei einer Temperatur von 50°C bis 250°C, gegebenenfalls unter erhöhtem Druck, innerhalb einer Zeit von weniger als 10 Sekunden, und sofort darauf folgendes Mischen der Lösung mit einem wässrigen Medium bei einer Temperatur von 0 bis 90°C, wobei das Mischen mit dem wässrigen Medium in Gegenwart des mindestens einen physiologisch verträglichen Emulgators erfolgt.

2. Verfahren nach Anspruch 1, **dadurch gekennzeichnet, daß** die Teilchengröße in der Carotinoid- oder Retinoidsuspension kleiner als 1 µm ist.

3. Verfahren nach Anspruch 1 oder 2, **dadurch gekennzeichnet, daß** das mit Wasser mischbare flüchtige Lösungsmittel mindestens ein Alkohol, Keton, Ester, Acetal oder Ether oder ein Gemisch von einem oder mehreren davon ist.

4. Verfahren nach Anspruch 3, **dadurch gekennzeichnet, daß** das mit Wasser mischbare flüchtige Lösungsmittel Aceton, 1,2-Butandiol-1-methylether, 1,2-Propandiol-1-n-propylether, Ethanol, n-Propanol, Isopropanol oder ein Gemisch von zweien oder mehreren davon ist.

5. Verfahren nach einem der Ansprüche 1 bis 4, **dadurch gekennzeichnet, daß** das Carotinoid oder Retinoid beim Lösen in Form einer Suspension in einem flüchtigen, mit Wasser mischbaren organischen Verdünnungs- oder Lösungsmittel eingesetzt wird, vorzugsweise in dem beim Lösen verwendeten Lösungsmittel.

6. Verfahren nach einem der Ansprüche 1 bis 5, **dadurch gekennzeichnet, daß** das Lösen des Carotinoids oder Retinoids im flüchtigen, mit Wasser mischbaren organischen Lösungsmittel in einer Mischkammer erfolgt und das Mischen der Lösung mit einem wässrigen Medium in einer mit der ersten Mischkammer in Reihe geschalteten zweiten Mischkammer erfolgt und das Verfahren wahlweise kontinuierlich durchgeführt wird.

7. Verfahren nach einem der Ansprüche 1 bis 6, **dadurch gekennzeichnet, daß** die Konzentration des Carotinoids oder Retinoids in der Suspension 0,1 bis 100 g/l beträgt.

8. Verfahren nach einem der Ansprüche 1 bis 7, **dadurch gekennzeichnet, daß** das Gewichtsverhältnis von Emulgator zu Carotinoid oder Retinoid in der Suspension 0,1 bis 5 beträgt.

9. Verfahren nach einem der Ansprüche 1 bis 8, **dadurch gekennzeichnet, daß** das Wasser mischbare organische Lösungsmittel oder gegebenenfalls die Suspension des Carotinoids oder Retinoids als Antioxidationsmittel Tocopherol, t-Butylhydroxytoluol, t-Butylhydroxyanisol oder Ethoxyquine enthält.

10. Verfahren zur Herstellung eines feinverteilten Carotinoid- oder Retinoidpulvers, **dadurch gekennzeichnet, daß** man eine Carotinoid- oder Retinoidsuspension durch ein Verfahren gemäß einem der Ansprüche 1 bis 9 herstellt und die Carotinoid- oder Retinoidsuspension sprühtrocknet zu einem feinverteilten Pulver.

11. Carotinoid- oder Retinoidsuspension mit einer Teilchengröße kleiner 1 µm in einem wasserhaltigen Medium, **dadurch gekennzeichnet, daß** die Suspension besteht aus Wasser, einem flüchtigen, mit Wasser mischbaren organischen Lösungsmittel, einem Carotinoid oder Retinoid, mindestens einem physiologisch verträglichen Emulgator, ausgewählt aus Lecithin, einem Fettsäuresalz, einem Mono-, Di- oder Triglycerid von C₁₂-C₁₈-Fettsäuren oder aliphatischen, gegebenenfalls acetylierten, Polycarbonsäuren, gegebenenfalls verestert mit Fruchtsäuren, einem Zuckerfettsäureester, oder einem Polyglycerinester von C₁₂-C₁₈-Fettsäuren, und gegebenenfalls einem Antioxidationsmittel.

12. Suspension nach Anspruch 11, **dadurch gekennzeichnet, daß** die Konzentration des Carotinoids oder Retinoids in der Suspension 0,1 bis 100 g/l beträgt.

13. Suspension nach Anspruch 11 oder 12, **dadurch gekennzeichnet, daß** das Gewichtsverhältnis von Emulgator zu Carotinoid oder Retinoid in der Suspension 0,1 bis 5 beträgt.

14. Verwendung der Carotinoidsuspension gemäß einem der Ansprüche 11 bis 13 oder des gemäß Anspruch 10 erhaltenen feinverteilten Carotinoidpulvers als Färbemittel für Lebens- und Futtermittel.

## Claims

1. A process for preparing finely dispersed carotenoid or retinoid suspensions consisting of water, a volatile, water-miscible organic solvent, a carotenoid or retinoid, at least one physiologically tolerated emulsifier selected from lecithin, a fatty acid salt, a mono-, di- or triglyceride of C₁₂-C₁₈-fatty acids or aliphatic, where appropriate acetylated, polycarboxylic acids, where appropriate esterified with fruit acids, a sugar fatty acid ester, or a polyglycerol ester of C₁₂-C₁₈-fatty acids, and, where appropriate, an antioxidant by dissolving the carotenoid or retinoid in a volatile, water-miscible organic solvent at 50°C to 250°C, where appropriate under elevated pressure, within less than 10 seconds, and immediately thereafter mixing the solution with an aqueous medium at from 0 to 90°C, where the mixing with the aqueous medium takes place in the presence of at least one physiologically tolerated emulsifier.

2. A process as claimed in claim 1, wherein the particle size in the carotenoid or retinoid suspension is less than 1 µm.

3. A process as claimed in claim 1 or 2, wherein the water-miscible volatile solvent is at least one alcohol, ketone, ester, acetal or ether or a mixture of one or more thereof.

4. A process as claimed in claim 3, wherein the water-miscible volatile solvent is acetone, 1,2-butanediol 1-methyl ether, 1,2-propanediol 1-n-propyl ether, ethanol, n-propanol, isopropanol or a mixture of two or more thereof.

5. A process as claimed in any of the claims 1 to 4, wherein the carotenoid or retinoid is employed in the dissolving in the form of a suspension in a volatile, water-miscible organic diluent or solvent, preferably in the solvent used in the dissolving.

6. A process as claimed in any of claims 1 to 5, wherein the dissolving of the carotenoid or retinoid in the volatile, water-miscible organic solvent takes place in a mixing chamber, and the mixing of the solution with an aqueous medium takes place in a second mixing chamber which is connected in series with the first mixing chamber, and the process is chosen to be carried out continuously.

7. A process as claimed in any of claims 1 to 6, wherein the concentration of the carotenoid or retinoid in the suspension is from 0.1 to 100 g/l.

8. A process as claimed in any of claims 1 to 7, wherein the ratio of emulsifier to carotenoid or retinoid in the suspension is from 0.1 to 5 by weight.

9. A process as claimed in any of claims 1 to 8, wherein the water-miscible organic solvent or, where appropriate, the suspension of the carotenoid or retinoid contains tocopherol, t-butylhydroxytoluene, t-butylhydroxyanisole or ethoxyquin as antioxidant.

10. A process for preparing a finely dispersed carotenoid or retinoid powder, which comprises preparing a carotenoid or retinoid suspension by a process as claimed in any of claims 1 to 9, and spray drying the carotenoid or retinoid suspension to give a finely dispersed powder.

11. A carotenoid or retinoid suspension with a particle size of less than 1 _{µ}m in a water-containing medium, wherein the suspension consists of water, a volatile, water-miscible organic solvent, a carotenoid or retinoid, at least one physiologically tolerated emulsifier selected from lecithin, a fatty acid salt, a mono-, di- or triglyceride of C₁₂-C₁₈-fatty acids or aliphatic, where appropriate acetylated, polycarboxylic acids, where appropriate esterified with fruit acids, a sugar fatty acid ester, or a polyglycerol ester of C₁₂-C₁₈-fatty acids, and, where appropriate, an antioxidant.

12. A suspension as claimed in claim 11, wherein the concentration of the carotenoid or retinoid in the suspension is from 0.1 to 100 g/l.

13. A suspension as claimed in claim 11 or 12, wherein the ratio of emulsifier to carotenoid or retinoid in the suspension is from 0.1 to 5 by weight.

14. The use of the carotenoid suspension as claimed in any of claims 11 to 13 or of the finely dispersed carotenoid powder as set forth in claim 10 as coloring agent for human and animal foods.

## Revendications

1. Procédé pour la préparation de suspensions de caroténoïdes ou de rétinoides finement divisés, consistant en de l'eau, un solvant organique volatil, miscible avec l'eau, un caroténoïde ou un rétinoïde, au moins un émulsifiant physiologiquement acceptable, choisi parmi la lécithine, un sel d'acide gras, un mono-, di- ou triglycéride d'acide gras en C₁₂-C₁₈ ou d'acides polycarboxyliques aliphatiques, éventuellement acétylés, estérifié en option avec des acides de fruit, un ester d'acide gras de sucre, ou un ester de polyglycérol d'acide gras en C₁₂-C₁₈, et éventuellement un antioxydant, par dissolution du caroténoïde ou du rétinoïde dans le solvant organique volatil, miscible avec l'eau, à une température de 50°C à 250°C, éventuellement sous pression élevée, sur une durée inférieure à 10 secondes, et aussitôt après mélange subséquent de la solution avec un milieu aqueux à une température de 0 à 90°C, tandis que le mélange avec le milieu aqueux est effectué en présence du ou des émulsifiants physiologiquement acceptables.

2. Procédé selon la revendication 1, **caractérisé par le fait que** la taille des particules est inférieure à 1 µm dans la suspension de caroténoide ou de retinoide.

3. Procédé selon la revendication 1 ou 2, **caractérisé par le fait que** le solvant volatil miscible avec l'eau est au moins un alcool, une cétone, un ester, un acétal ou un éther, ou un mélange de l'un ou plusieurs de ceux-ci.

4. Procédé selon la revendication 3, **caractérisé par le fait que** le solvant volatil miscible avec l'eau est l'acétone, l'éther 1,2-butanediol-1-méthylique, l'éther l,2-propanediol-1-n-propylique, l'éthanol, le n-propanol, l'isopropanol ou un mélange de deux ou plusieurs de ceux-ci.

5. Procédé selon l'une des revendications 1 à 4, **caractérisé par le fait que** le caroténoide ou le rétinoïde est utilisé lors de la dissolution sous forme d'une suspension dans un agent de dilution ou un solvant organique, volatil, miscible avec l'eau, de préférence dans le solvant utilisé lors de la dissolution.

6. Procédé selon l'une des revendications 1 à 5, **caractérisé par le fait que** la dissolution du caroténoïde ou du rétinoïde dans le solvant organique volatil, miscible avec l'eau s'effectue dans une chambre de mélange et le mélange de la solution avec un milieu aqueux s'effectue dans une deuxième chambre de mélange montée en série avec la première chambre de mélange et le procédé est mis en oeuvre en continu, en fonction des besoins.

7. Procédé selon l'une des revendications 1 à 6, **caractérisé par le fait que** la concentration du caroténoïde ou du rétinoïde dans la suspension est de 0,1 à 100 g/l.

8. Procédé selon l'une des revendications 1 à 7, **caractérisé par le fait que** le rapport en poids de l'émulsifiant au caroténoïde ou au rétinoïde dans la suspension est de 0,1 à 5.

9. Procédé selon l'une des revendications 1 à 8, **caractérisé par le fait que** le solvant organique miscible à l'eau ou éventuellement la suspension du caroténoïde ou du rétinoïde contient comme antioxydant du tocophérol, du t-butylhydroxytoluène, du t-butylhydroxyanisole ou de l'éthoxyquine.

10. Procédé pour la préparation d'une poudre de caroténoïde ou de rétinoïde finement divisée, **caractérisé par le fait qu'**on prépare une suspension de caroténoïde ou de rétinoïde par un procédé selon l'une des revendications 1 à 9 et on sèche par pulvérisation la suspension de caroténoïde ou de rétinoïde pour former une poudre finement divisée.

11. Suspension de caroténoïde ou de rétinoïde ayant une taille de particules inférieure à 1 µm dans un milieu contenant de l'eau, **caractérisée par le fait que** la suspension consiste en de l'eau, un solvant organique volatil, miscible avec l'eau, un caroténoïde ou un rétinoïde, au moins un émulsifiant physiologiquement acceptable, choisi parmi la lécithine, un sel d'acide gras, un mono-, di- ou triglycéride d'acide gras en C₁₂-C₁₈ ou des acides polycarboxyliques aliphatiques, éventuellement acétylés, en option estérifié, avec des acides de fruit, un ester d'acide gras de sucre, ou un ester de polyglycérol d'acide gras en C₁₂-C₁₈ et éventuellement un antioxydant.

12. Suspension selon la revendication 11, **caractérisée par le fait que** la concentration de caroténoïde ou de rétinoïde dans la suspension est de 0,1 à 100 g/l.

13. Suspension selon la revendication 11 ou 12, **caractérisée par le fait que** le rapport en poids de l'émulsifiant au caroténoïde ou au rétinoïde dans la suspension est de 0,1 5.

14. Utilisation de la suspension de caroténoïde selon l'une des revendications 11 à 13 ou de la poudre de caroténoïde finement divisée obtenue selon la revendication 10 comme colorant pour des denrées alimentaires et des aliments pour le bétail.
